# EUROPEAN PATENT SPECIFICATION

(11) **EP 3 232 942 B1**
(45) Date of publication and mention of the grant of the patent: **22.02.2023**
(21) Application number: 15775572.9
(22) Date of filing: 25.09.2015
(51) Int. Cl.: A61B 17/04, A61B 17/86, A61B 17/80, A61F 2/08, A61L 31/14

(54) **SURGICAL IMPLANT WITH POROUS REGION**
CHIRURGISCHES IMPLANTAT MIT PORÖSER REGION
IMPLANT CHIRURGICAL AVEC RÉGION POREUSE

(30) Priority: 16.12.2014 US 201414571677
(43) Date of publication of application: 25.10.2017
(73) Proprietor: Arthrex, Inc., Naples, FL 34108-1945 (US)
(72) Inventor: PAPANGELOU, Chris, Bonita Springs, Florida 34135 (US); KARNES, G. Joshua, Estero, Florida 33928 (US)
(74) Representative: Mewburn Ellis LLP
(86) International application number: PCT/US2015/052121
(87) International publication number: WO 2016/099620

(56) References cited:
- WO-A1-2012/109748
- WO-A1-2014/068259
- US-A1- 2006 111 715
- US-A1- 2006 276 788
- US-A1- 2008 269 893
- US-A1- 2010 042 214
- US-A1- 2010 094 420
- US-A1- 2013 022 943

## Description

### CROSS REFERENCE TO RELATED APPLICATION

This application claims priority to US Patent Application Serial No. 14/571,677 filed December 16, 2014.

### BACKGROUND

This disclosure relates to a surgical implant. The surgical implant includes at least one porous region that facilitates tissue ingrowth through the implant.

Orthopedic procedures are often performed to repair musculoskeletal injuries. For example, soft tissue may tear away from bone during vigorous exercise or sporting activities. When tears occur, reattachment may be necessary to repair the damaged tissue. Various surgical implants can be used to repair damaged tissue, including but not limited to screws, anchors, wedges, etc.

Many surgical implants are made from bio-absorbable materials. The bio-absorbable materials must be of sufficient strength to withstand loads encountered during the healing process and must have a chemistry that is compatible with the human body.
US 2013/022943 A1 discloses a dental implant that comprises a shaft defining a longitudinal axis and having an apical end, a coronal end, and an exterior surface. A portion of the exterior surface can include a porous material. The dental implant can comprise at least one thread, including a non-porous material, having an interior surface and a non-engaging surface. The interior surface can engage and wind around the exterior surface of the shaft and the bone-engaging surface can extend outwardly from the exterior surface of the shaft.
US 2010/042214 A1 discloses an orthopaedic screw that provides for immediate delivery of a therapeutic agent through channels and/or holes and reservoirs for longterm delivery of a therapeutic agent. The porous nature of the orthopaedic screw and the ability to deliver therapeutic agents to the surrounding tissue can impact tissue integration.
US 2006/276788 A1 discloses a bone screw construction having a porous structure located along a portion of a shaft that contains bone engaging threads. The bone threads are constructed of generally dense material of high mechanical strength. Along a minor diameter of the thread form is located a spiral-shaped porous structure which wraps around the body or shank of the screw for the entire thread length. This allows for a continuous thread form to extend along the screw length from the point to the head and creates and unbroken porous ingrowth structure along that same length of the screw. Further implants are known from US 2006/111715 A1, US 2010/094420 A1, US 2008/269893 A1, WO 2014/068259 A1, and WO 2012/109748 A1.

### SUMMARY

The present invention is defined by appended claim 1. Specific embodiments are set forth in the dependent claims.

A surgical implant according to an exemplary aspect of the present disclosure includes, among other things, a body that extends along a longitudinal axis between a drive head and a tip. The body includes a cannulation disposed about the longitudinal axis and extending from the tip to the drive head, a solid thread that wraps around the body and a porous region circumferentially extending about the body between adjacent revolutions of the solid thread.

In a further non-limiting embodiment of the foregoing surgical implant, the surgical implant is a suture anchor.

In a further non-limiting embodiment of either of the foregoing surgical implants, the surgical implant is an interference screw.

In a further non-limiting embodiment of any of the foregoing surgical implants the porous region includes a plurality of interconnected pores.

In a further non-limiting embodiment of any of the foregoing surgical implants, the tip and the drive head are solid.

In a further non-limiting embodiment of any of the foregoing surgical implants, a porous extension extends from the porous region to the cannulation.

In a further non-limiting embodiment of any of the foregoing surgical implants, a vent connects between the cannulation and the porous region.

In a further non-limiting embodiment of any of the foregoing surgical implants, the body includes an inner wall that circumscribes the cannulation, the inner wall being solid.

In a further non-limiting embodiment of any of the foregoing surgical implants, a solid strut connects between the solid thread and the cannulation.

In a further non-limiting embodiment of any of the foregoing surgical implants, the solid thread includes a minor diameter and a major diameter, and the solid thread is completely solid between the minor diameter and the major diameter.

In a further non-limiting embodiment of any of the foregoing surgical implants, the body and the porous region are made of the same material.

In a further non-limiting embodiment of any of the foregoing surgical implants, the porous region is disposed in a portion of the body that extends between the cannulation and the solid thread.

A surgical implant according to another exemplary aspect of the present disclosure includes, among other things, a body that extends along a longitudinal axis between a drive head and a tip. The body includes a cannulation disposed along the longitudinal axis and extending from the tip to the drive head, the cannulation circumscribed by an inner wall. A solid thread spirals about the body between the drive head and the tip, the solid thread being completely solid between a minor diameter and a major diameter. A porous region includes a plurality of interconnected pores that are formed between adjacent revolutions of the solid thread. A solid strut connects between the solid thread and the inner wall.

In a further non-limiting embodiment of the foregoing surgical implant, a vent is formed through the inner wall.

In a further non-limiting embodiment of either of the foregoing surgical implants, the porous region includes a porous extension that extends to the inner wall.

In a further non-limiting embodiment of any of the foregoing surgical implants, the porous region includes a height that extends across at least two of the revolutions of the solid thread.

A surgical implant according to another exemplary aspect of the present disclosure includes, among other things, a body extending between an inner wall an outer wall and at least one porous region that extends continuously around the body between the inner wall and the outer wall.

In a further non-limiting embodiment of the foregoing surgical implant, the surgical implant is a wedge that includes a plurality of porous regions disposed in the body. Each porous region of the plurality of porous regions connects to an adjacent porous region of the plurality of porous regions.

In a further non-limiting embodiment of either of the foregoing surgical implants, the plurality of porous regions extend continuously through the body to establish an uninterrupted network of interconnected pores inside the body.

In a further non-limiting embodiment of any of the foregoing surgical implants, the plurality of porous regions connect to one another through vents formed in the inner wall, the outer wall and solid struts of the body.

The embodiments, examples and alternatives of the preceding paragraphs, the claims, or the following description and drawings, including any of their various aspects or respective individual features, may be taken independently or in any combination. Features described in connection with one embodiment are applicable to all embodiments, unless such features are incompatible.

Accordingly, a surgical implant according to an exemplary aspect of the present disclosure includes a body that extends along a longitudinal axis between a drive head and a tip, a cannulation in the body disposed about the longitudinal axis and extending from the tip to the drive head, a solid thread that wraps around the body, and a porous region circumferentially extending about the body between adjacent revolutions of the solid thread.

A surgical implant may also include a porous extension that extends from porous region to the cannulation. A surgical implant may also include a vent that connects between the cannulation and porous region. A surgical implant may also include a solid strut that connects between the solid thread and the cannulation. A surgical implant may also include a porous extension and a vent. A surgical implant may also include a porous extension and/or a vent, and a solid strut.

In a surgical implant also having any one or more of these further features, the porous region may include a plurality of interconnected pores and may be disposed in a portion of the body that extends between the cannulation and the solid thread, the tip and the drive head may be solid, the porous region may include a height that extends across at least two of the revolutions of the solid thread, the body may include a solid inner wall that circumscribes the cannulation, and the solid thread may be completely solid between a minor diameter and a major diameter of the solid thread. For example, the surgical implant also includes a porous extension and/or a vent, a solid strut, and the porous region includes a plurality of interconnected pores disposed in a portion of the body that extends between the cannulation and the solid thread, the porous region includes a height that extends across at least two of the revolutions of the solid thread, the tip and the drive head are solid, the body includes a solid inner wall that circumscribes the cannulation, and the solid thread is completely solid between a minor diameter and a major diameter of the solid thread.

In a surgical implant also having any one or more of these further features, the body and the porous region may be made of the same material.

A surgical implant also having any one or more of these further features may be a suture anchor or an interference screw.

A surgical implant according to another exemplary aspect of the present disclosure includes a body that extends along a longitudinal axis between a drive head and a tip, a cannulation in the body that is disposed along the longitudinal axis and extending from the tip to the drive head and is circumscribed by an inner wall, a solid thread that spirals about the body between the drive head and the tip and is completely solid between a minor diameter and a major diameter of the solid thread, a porous region including a plurality of interconnected pores that are formed between adjacent revolutions of the solid thread, and a solid strut that connects between the solid thread and the inner wall.

A surgical implant may also include a vent formed through the inner wall. The surgical implant may also include a porous extension that extends from the porous region to the inner wall. A surgical implant may also include a vent and a porous extension.

In a surgical implant also having any one or more of these further features, the porous region may be disposed in a portion of the body that extends between the cannulation and the solid thread, the porous region may include a height that extends across at least two of the revolutions of the solid thread, and the tip and the drive head may be solid. For example, the surgical implant also includes a vent and/or a porous extension, the porous region is disposed in a portion of the body that extends between the cannulation and the solid thread, the porous region includes a height that extends across at least two of the revolutions of the solid thread, and the tip and the drive head are solid.

In a surgical implant also having any one or more of these further features, the body and the porous region may be made of the same material.

A surgical implant also having any one or more of these further features may be a suture anchor or an interference screw.

A surgical implant according to another exemplary aspect of the present disclosure includes a body extending between an inner wall and an outer wall and at least one porous region that extends continuously around the body between the inner wall and the outer wall.

A surgical implant may be a wedge that includes a plurality of porous regions disposed in the body. Each porous region of the plurality of porous regions connects to an adjacent porous region of the plurality of porous regions. A porous region may include a plurality of interconnected pores.

In a surgical implant having a plurality of porous regions, the plurality of porous regions may extend continuously through the body to establish an uninterrupted network of interconnected pores inside the body. In a surgical implant having a plurality of porous regions, the plurality of porous regions may connect to one another through vents formed in the inner wall, the outer wall and solid struts of the body. In a surgical implant having a plurality of porous regions, the plurality of porous regions may extend continuously through the body to establish an uninterrupted network of interconnected pores inside the body and may connect to one another through vents formed in the inner wall, the outer wall and solid struts of the body. For example, surgical implant is a wedge that includes a plurality of porous regions disposed in the body, each porous region of the plurality of porous regions connecting to an adjacent porous region of the plurality of porous regions, and the plurality of porous regions extend continuously through the body to establish an uninterrupted network of interconnected pores inside the body and/or connect to one another through vents formed in the inner wall, the outer wall and solid struts of the body.

In a surgical implant also having any one or more of these further features, the body and the porous region may be made of the same material.

Aspects, features and embodiments of the present disclosure are described further below.

For example, an exemplary surgical implant of the present disclosure includes a body that extends along a longitudinal axis between a solid (i.e., substantially non-porous) drive head and a solid (i.e., substantially non-porous) tip, a cannulation in the body that extends from the tip to the drive head, a solid (i.e., substantially non-porous) thread that wraps around the body between the drive head and the tip, and one or more porous regions formed in the body. The surgical implant may also include one or more solid (i.e., substantially non-porous) struts connecting solid sections of the implant.

A surgical implant is configured for use in various soft tissue repairs and may be fixated within bone to attach tissue (e.g., ligament, tendon, graft, etc.) to the bone. For example, the surgical implant is an interference screw that can be used in conjunction with a variety of orthopedic surgical repairs, such as an ACL reconstruction. The cannulation that extends from the tip to the drive head establishes a passage through the body for accommodating a guide pin, wire or shaft. The solid thread includes a minor diameter D1 and a major diameter D2. Each porous region includes a plurality of interconnected pores. The porous regions establish areas of increased porosity that facilitate tissue ingrowth subsequent to implantation of the surgical implant into bone. The solid struts increase the strength and stability of the body of the surgical implant near the porous regions.

The body may or may not taper between the drive head and the tip. The body may be of any size and shape suitable for the intended use of the surgical implant. The drive head may accommodate a driver for insertion of the surgical implant into bone. For example, the drive head may include a hex opening for receiving a corresponding hex-head of a driver or could include configurations other than the hex shape. The tip may be shaped in any manner to facilitate insertion of the surgical implant into bone, for example, the tip may be blunted, rounded, pointed, or otherwise shaped. The cannulation may be circumferentially disposed about the longitudinal axis. The solid thread may wrap around the body in a helical pattern. For example, the solid thread may spiral around the body and includes a plurality of revolutions. The dimension of the minor diameter D1 and the major diameter D2 of the solid thread, as well as thread pitch and other properties associated with the solid thread, may be any suitable for the intended use of the implant. The solid thread may be either a continuous or a discontinuous thread. The solid thread may be completely solid, or substantially non-porous, between the minor diameter D1 and the major diameter D2. The porous regions may be provided in selected regions of the body. For example, the porous regions may circumferentially extend about the body between adjacent revolutions of the solid thread. For example, the porous regions are formed in the body between the cannulation and the solid thread. For example, the porous regions extend from the minor diameter D1 toward the cannulation. Some of the porous regions may include a height H that extends across multiple revolutions of the solid thread. The plurality of interconnected pores of the porous region can be controlled to any desired size and shape and may be formed at any desired location within the surgical implant to facilitate tissue ingrowth. The solid struts may extend between the solid thread and the cannulation. The solid struts may be generally cylindrical shaped. The solid struts may extend from an inner wall of the body that circumscribes the cannulation to the solid thread. The porous regions may include a porous extension that extends through the inner wall of the body which circumscribes the cannulation for further facilitating tissue ingrowth.

A surgical implant may be made of a bio-absorbable polymer. The body and the porous regions of the surgical implant may be made of the same material.

Non-limiting examples of suitable materials for the surgical implant include poly-L-lactide (PLLA), poly lactic acid (PLDLA), PLLA with biphasic calcium phosphate, or PLDLA with hydroxyapatite plus biphasic calcium phosphate. Other exemplary materials may include thermosets, thermoplastics, bio-absorbable materials comprised of a single polymer, blend, or composites made of multiple materials, multiple polymers, fibers with polymer or granules/powder with polymer. Other materials are also contemplated within the scope of this disclosure.

Porous regions may be formed in bio-absorbable polymer that makes up a surgical implant using a variety of techniques. In one non-limiting technique, the porous regions are formed by merging granules of polyvinyl alcohol (PVA) with the bio-absorbable polymer, curing the PVA together with the bio-absorbable polymer, and then dissolving the PVA granules out of the bio-absorbable polymer. Other techniques for forming the porous regions may alternatively or additionally be utilized.

In another example, an exemplary surgical implant of the present disclosure includes a body that extends along a longitudinal axis between a solid (i.e., substantially non-porous) drive head and a solid (i.e., substantially non-porous) tip, a cannulation that extends from the tip to the drive head, a solid (i.e., substantially non-porous) thread that wraps around the body between the drive head and the tip which is completely solid between a minor diameter D1 and a major diameter D2 of the solid thread, and one or more porous regions formed in the body. The surgical implant may also include one or more solid (i.e., substantially non-porous) struts connecting solid sections of the surgical implant. The surgical implant may also include one or more vents formed through an inner wall of the body that circumscribes the cannulation.

A surgical implant is configured for use to attach tissue to bone as part of a rotator cuff repair, Achilles tendon repair, patellar tendon repair, ACL/PCL reconstruction, hip and shoulder reconstructions, or any other orthopedic repair. For example, the surgical implant is a suture anchor that can be used to attach tissue to bone as part of a rotator cuff repair, Achilles tendon repair, patellar tendon repair, ACL/PCL reconstruction, hip and shoulder reconstructions, or any other orthopedic repair. The cannulation that extends from the tip to the drive head establishes a passage through the body for accommodating a shaft, which may be part of a driver, for example. Each porous region includes a plurality of interconnected pores. The porous regions establish areas of increased porosity that facilitate tissue ingrowth subsequent to implantation of the surgical implant into bone. The solid struts increase the strength and stability of the body of the surgical implant near the porous regions. The vents facilitate tissue ingrowth through the surgical implant.

The body may or may not taper between the drive head and the tip. The body may be of any size and shape suitable for the intended use of the surgical implant. The drive head may accommodate a driver for insertion of the surgical implant into bone. For example, the drive head may include a hex opening for receiving a corresponding hex-head of a driver or could include configurations other than the hex shape. The tip may be shaped in any manner to facilitate insertion of the surgical implant into bone, for example, the tip may be blunted, rounded, pointed, or otherwise shaped. The cannulation may be circumferentially disposed about the longitudinal axis. The solid thread may wrap around the body in a helical pattern. For example, the solid thread may spiral around the body and includes a plurality of revolutions. The dimension of the minor diameter D1 and the major diameter D2 of the solid thread, as well as thread pitch and other properties associated with the solid thread, may be any suitable for the intended use of the implant. The solid thread may be either a continuous or a discontinuous thread. The porous regions may be provided in selected regions of the body. For example, the porous regions may circumferentially extend about the body between adjacent revolutions of the solid thread. For example, the porous regions are formed in the body between the cannulation and the solid thread. For example, the porous regions extend from the minor diameter D1 toward the cannulation. Some of the porous regions may include a height H that extends across multiple revolutions of the solid thread. The plurality of interconnected pores of the porous region can be controlled to any desired size and shape and may be formed at any desired location within the surgical implant to facilitate tissue ingrowth. The solid struts may extend between the solid thread and the cannulation. The solid struts may be generally cylindrical shaped. The solid struts may extend between the solid thread and an inner wall of the body that circumscribes the cannulation. The porous regions may include a porous extension that extends through the inner wall of the body which circumscribes the cannulation for further facilitating tissue ingrowth.

The surgical implant may be made of a bio-absorbable polymer. The body and the porous regions of the surgical implant may be made of the same material.

Non-limiting examples of suitable materials for the surgical implant include poly-L-lactide (PLLA), poly lactic acid (PLDLA), PLLA with biphasic calcium phosphate, or PLDLA with hydroxyapatite plus biphasic calcium phosphate. Other exemplary materials may include thermosets, thermoplastics, bio-absorbable materials comprised of a single polymer, blend, or composites made of multiple materials, multiple polymers, fibers with polymer or granules/powder with polymer. Other materials are also contemplated within the scope of this disclosure.

Porous regions may be formed in the bio-absorbable polymer that makes up a surgical implant using a variety of techniques. In one non-limiting technique, the porous regions are formed by merging granules of polyvinyl alcohol (PVA) with the bio-absorbable polymer, curing the PVA together with the bio-absorbable polymer, and then dissolving the PVA granules out of the bio-absorbable polymer. Other techniques for forming the porous regions may alternatively or additionally be utilized.

In another example, an exemplary surgical implant of the present disclosure includes a body that extends between an inner wall and an outer wall that is spaced from the inner wall, and one or more porous regions formed in the body. The surgical implant may also include a central bore that extends through the body inside of the inner wall. The surgical implant may also include a top face and a bottom face of the body that extend between the inner wall and the outer wall. The surgical implant may also include one or more solid struts connecting between solid sections of the surgical implant. The surgical implant may also include vents, or openings, formed through both the inner wall and the outer wall. The surgical implant may also include vents that extend through the solid struts.

A surgical implant is configured for use to repair a bone deformity. For example, the surgical implant is a wedge, such as an osteotomy wedge, that can be used to repair a bone deformity. For example, the surgical implant can be implanted during an osteotomy procedure to repair a deformity associated with a knee, ankle, foot or other joint. Each porous region includes a plurality of interconnected pores that establish areas of increased porosity that facilitate tissue ingrowth subsequent to implantation of the surgical implant into bone. The central bore may further facilitate tissue ingrowth. The solid struts increase the strength and stability of the body of the surgical implant near the porous regions. The vents facilitate tissue ingrowth through the surgical implant.

The body may be of any size and any shape suitable for the intended use of the surgical implant. The outer wall may circumscribe the inner wall. Porous regions may be provided in selected regions of the body. For example, at least one continuous porous region extends continuously around the body between the inner wall and the outer wall. For example, some of the porous regions may extend through the inner wall and/or the outer wall. The plurality of interconnected pores of the porous region can be controlled to any desired size and shape and may be formed at any desired location within the surgical implant to facilitate tissue ingrowth. The solid struts may extend between solid portions of the inner wall and the outer wall and between solid portions of the top face and the bottom face. The porous regions may be at least partially separated from one another by the solid struts. Adjacent porous regions of the surgical implant may connect with one another via the vents to establish a continuous area of porosity distributed through the entirety of the body. Stated another way, the porous regions may extend continuously through the body to establish an uninterrupted network of interconnected pores inside the body. Therefore, the porous regions may not be discrete regions of the body that are isolated from other porous regions.

A surgical implant may be made of a bio-absorbable polymer. The body and the porous regions of the surgical implant may be made of the same material.

Non-limiting examples of suitable materials for the surgical implant include poly-L-lactide (PLLA), poly lactic acid (PLDLA), PLLA with biphasic calcium phosphate, or PLDLA with hydroxyapatite plus biphasic calcium phosphate. Other exemplary materials may include thermosets, thermoplastics, bio-absorbable materials comprised of a single polymer, blend, or composites made of multiple materials, multiple polymers, fibers with polymer or granules/powder with polymer. Other materials are also contemplated within the scope of this disclosure.

Porous regions may be formed in the bio-absorbable polymer that makes up the surgical implant using a variety of techniques. In one non-limiting technique, the porous regions are formed by merging granules of polyvinyl alcohol (PVA) with the bio-absorbable polymer, curing the PVA together with the bio-absorbable polymer, and then dissolving the PVA granules out of the bio-absorbable polymer. Other techniques for forming the porous regions may alternatively or additionally be utilized.

Porous regions of the various foregoing surgical implants are exemplary only and are not intended to limit this disclosure. The surgical implants may be designed to include more or less porosity within the scope of this disclosure.

As is apparent to those skilled in the art from the foregoing disclosure, while different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

The various features and advantages of this disclosure will become apparent to those skilled in the art from the following detailed description. The drawings that accompany the detailed description can be briefly described as follows.

### BRIEF DESCRIPTION OF THE DRAWINGS

Figure 1 illustrates a surgical implant according to a first embodiment of the invention.
Figure 2 is a cross-sectional view of the surgical implant of Figure 1.
Figure 3 illustrates the surgical implant of Figure 1 with the porous regions of the implant removed.
Figure 4 illustrates a magnified view of a porous region of a surgical implant.
Figure 5 illustrates a surgical implant according to a second embodiment of the invention.
Figure 6 is a cross-sectional view of the surgical implant of Figure 5.
Figures 7 and 8 illustrate the surgical implant of Figure 5 with the porous regions of the implant removed.
Figure 9 illustrates a surgical implant according to an example not forming part of the invention.
Figure 10 illustrates the surgical implant of Figure 9 with the porous regions of the implant removed.
Figure 11 is a cross-sectional view of the surgical implant of Figure 10.

### DETAILED DESCRIPTION

This disclosure describes surgical implants, such as screws, suture anchors, or wedges, which can be used to repair musculoskeletal injuries. The surgical implants include porous regions that facilitate tissue ingrowth subsequent to fixation of the implant within bone. In some embodiments, the surgical implant includes solid struts that increase the strength and stability of the implant near the porous regions. In other embodiments, the surgical implant includes vents formed through walls of the implant. These and other features of this disclosure are described in greater detail in the paragraphs that follow.

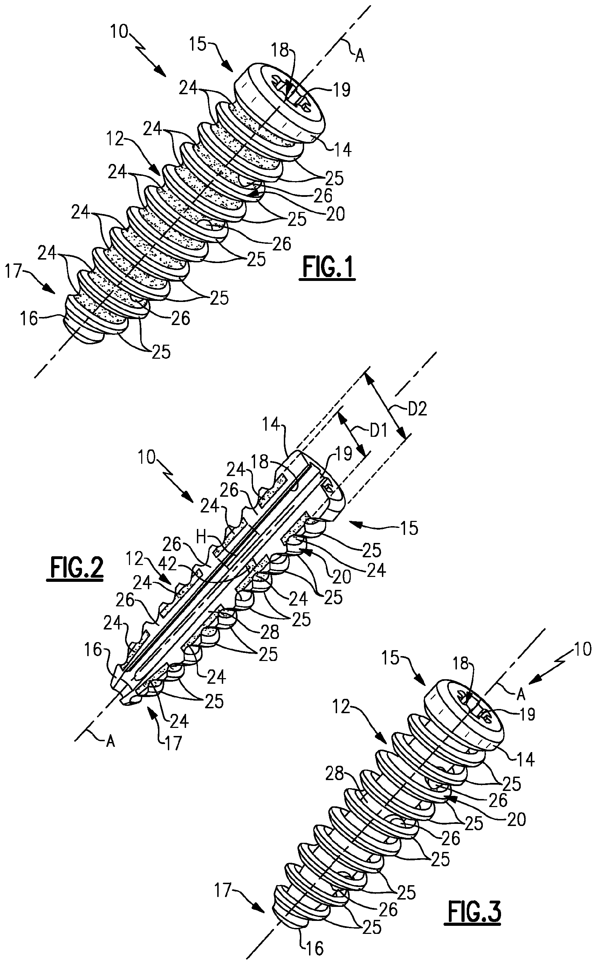

A surgical implant 10 is illustrated in Figures 1, 2 and 3. The surgical implant 10 is configured for use in various soft tissue repairs and may be fixated within bone to attach tissue (e.g., ligament, tendon, graft, etc.) to the bone. In one non-limiting embodiment, the surgical implant 10 is an interference screw that can be used in conjunction with a variety of orthopedic surgical repairs, such as an ACL reconstruction. Of course, this disclosure is not limited to interference screws or to ACL reconstructions and could have various additional implementations within the scope of this disclosure.

The exemplary surgical implant 10 includes a body 12 that extends along a longitudinal axis A between a drive head 14, formed at a proximal end 15, and a tip 16, formed at a distal end 17. The drive head 14 and the tip 16 are both solid portions (i.e., substantially non-porous) of the surgical implant 10. The body 12 may or may not taper between the drive head 14 and the tip 16. The size and shape of the body 12 are not intended to limit this disclosure.

The drive head 14 may accommodate a driver for insertion of the surgical implant 10 into bone. In one non-limiting embodiment, the drive head 14 includes a hex opening 19 for receiving a corresponding hex-head of a driver (not shown). The drive head 14 could include other configurations and is not limited to the hex shape.

The tip 16 may be shaped in any manner to facilitate insertion of the surgical implant 10 into bone. For example, the tip 16 may be blunted, rounded, pointed, or otherwise shaped.

The body 12 additionally includes a cannulation 18 that extends from the tip 16 to the drive head 14 (best shown in Figure 2). The cannulation 18 may be circumferentially disposed about the longitudinal axis A. The cannulation 18 establishes a passage through the body 12 for accommodating a guide pin, wire or shaft.

A solid thread 20 wraps around the body 12, such as in a helical pattern, between the drive head 14 and the tip 16. In one embodiment, the solid thread 20 spirals around the body 12 and includes a plurality of revolutions 25. The solid thread 20 includes a minor diameter D1 and a major diameter D2 (see Figure 2). The dimension of the minor diameter D1 and the major diameter D2, as well as thread pitch and other properties associated with the solid thread 20, are not intended to limit this disclosure. The solid thread 20 could also be either a continuous or a discontinuous thread. According to the invention, the solid thread 20 is completely solid, or substantially non-porous, between the minor diameter D1 and the major diameter D2.

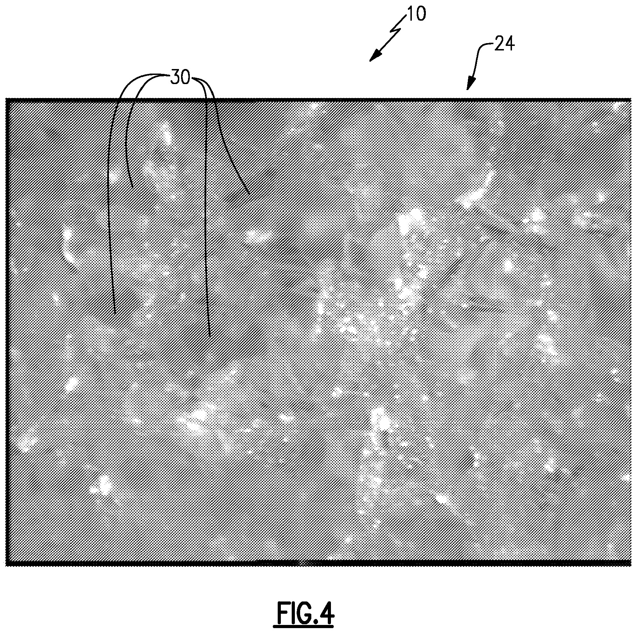

One or more porous regions 24 are formed in the body 12 of the surgical implant 10. Each porous region 24 includes a plurality of interconnected pores (see, for example, Figure 4). The porous regions 24 establish areas of increased porosity that facilitate tissue ingrowth subsequent to implantation of the surgical implant 10 into bone.

The porous regions 24 are provided in selected regions of the body 12. The porous regions 24 circumferentially extend about the body 12 between adjacent revolutions 25 of the solid thread 20. In one embodiment, the porous regions 24 are formed in the body 12 between the cannulation 18 and the solid thread 20. According to the invention, the porous regions 24 extend from the minor diameter D1 toward the cannulation 18. Some of the porous regions 24 may include a height H that extends across multiple revolutions 25 of the solid thread 20 (see Figure 2). Other configurations are also contemplated.

In another non-limiting embodiment, one or more solid struts 26 connect solid sections of the surgical implant 10. For example, the solid struts 26 may extend between the solid thread 20 and the cannulation 18. The solid struts 26 increase the strength and stability of the body 12 of the surgical implant 10 near the porous regions 24. In one embodiment, the solid struts 26 are generally cylindrical shaped.

In one embodiment, the surgical implant 10 may be made of a bio-absorbable polymer. Non-limiting examples of suitable materials include poly-L-lactide (PLLA), poly lactic acid (PLDLA), PLLA with biphasic calcium phosphate, or PLDLA with hydroxyapatite plus biphasic calcium phosphate. Other exemplary materials may include thermosets, thermoplastics, bio-absorbable materials comprised of a single polymer, blend, or composites made of multiple materials, multiple polymers, fibers with polymer or granules/powder with polymer. Other materials are also contemplated within the scope of this disclosure. In another embodiment, the body 12 and the porous regions 24 of the surgical implant are made of the same material.

The porous regions 24 may be formed in the bio-absorbable polymer that makes up the surgical implant 10 using a variety of techniques. In one non-limiting technique, the porous regions 24 are formed by merging granules of polyvinyl alcohol (PVA) with the bio-absorbable polymer, curing the PVA together with the bio-absorbable polymer, and then dissolving the PVA granules out of the bio-absorbable polymer. Other techniques for forming the porous regions 24 may alternatively or additionally be utilized.

Referring now to Figure 2 and Figure 3 (which illustrates the surgical implant 10 with the porous regions 24 removed for clarity), the body 12 may include an inner wall 28. The inner wall 28 circumscribes the cannulation 18. The solid struts 26 may extend from the inner wall 28 to the solid thread 20. In another embodiment, the porous regions 24 include a porous extension 42 that extends through the inner wall 28 for further facilitating tissue ingrowth (see, for example, Figure 2).

Figure 4 illustrates a magnified view of a porous region 24 of the surgical implant 10. The porous region 24 includes a plurality of interconnected pores 30. The interconnected pores 30 can be controlled to any desired size and shape and may be formed at any desired location within the surgical implant 10 to facilitate tissue ingrowth.

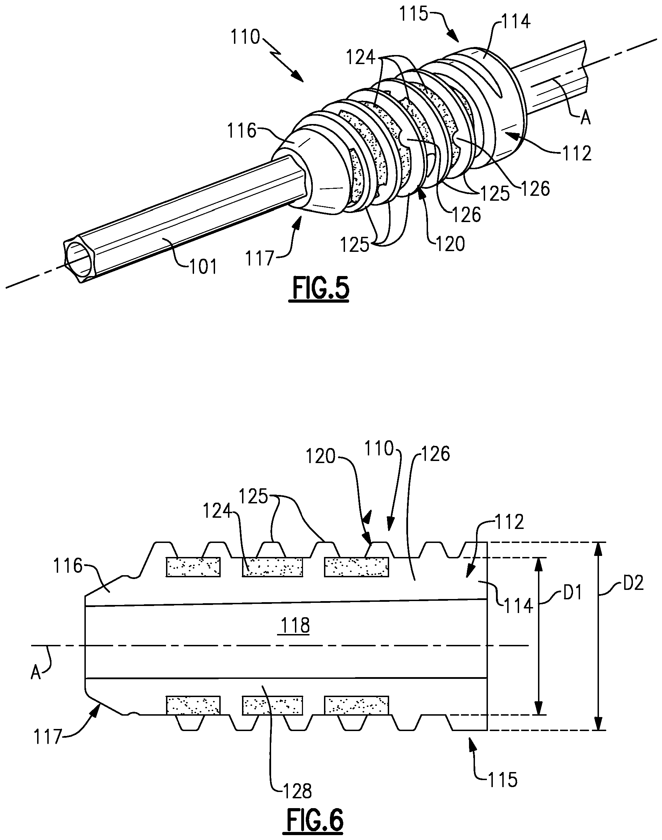

Figures 5 and 6 illustrate another surgical implant 110. In this disclosure, like reference numerals designate like elements where appropriate and reference numerals with the addition of 100 or multiples thereof designate modified elements that are understood to incorporate the same features and benefits of the corresponding original elements.

In this embodiment, the surgical implant 110 is a suture anchor that can be used to attach tissue to bone as part of a rotator cuff repair, Achilles tendon repair, patellar tendon repair, ACL/PCL reconstruction, hip and shoulder reconstructions, or any other orthopedic repair. The surgical implant 110 includes a body 112 that extends along a longitudinal axis A between a drive head 114, formed at a proximal end 115, and a tip 116, formed at a distal end 117.

A cannulation 118 (see Figure 6) extends from the tip 116 to the drive head 114. The cannulation 118 may be circumferentially disposed about the longitudinal axis A. The cannulation 118 establishes a passage through the body 112 for accommodating a shaft 101, which may be part of a driver, for example.

A solid thread 120 wraps around the body 112 between the drive head 114 and the tip 116. The solid thread 120 is completely solid between a minor diameter D1 and a major diameter D2 (see Figure 6). One or more porous regions 124 are formed in the body 112 of the surgical implant 110. The porous regions 124 circumferentially extend about the body 112 between adjacent revolutions 125 of the solid thread 120. In one embodiment, the porous regions 124 are formed in the body 112 between the cannulation 118 and the solid thread 120.

One or more solid struts 126 may connect solid sections of the surgical implant 110. For example, the solid struts 126 may extend between the solid thread 120 and an inner wall 128 of the body 112 that circumscribes the cannulation 118.

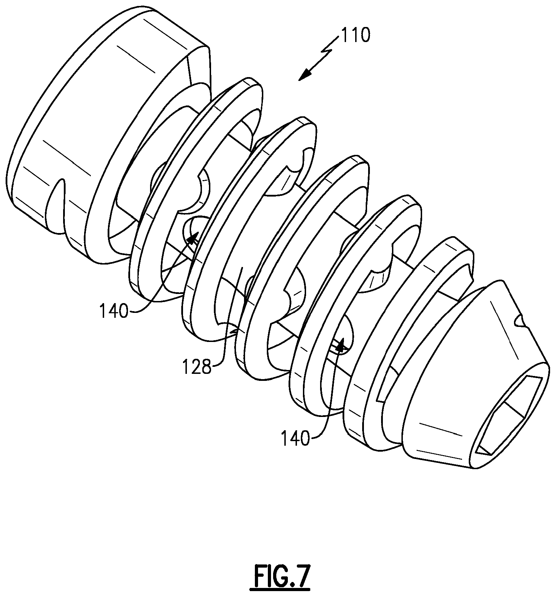

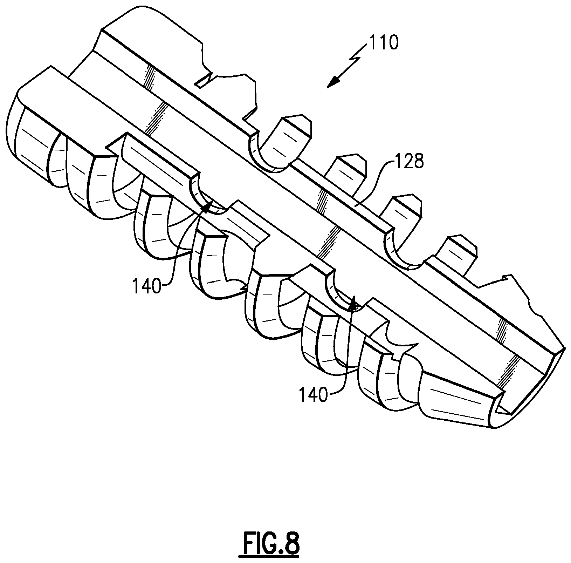

Referring to Figures 7 and 8, one or more vents 140 may be formed through the inner wall 128. The vents 140 may facilitate tissue ingrowth through the surgical implant 110. The porous regions 124 have been removed from Figures 7 and 8 to better illustrate the vents 140.

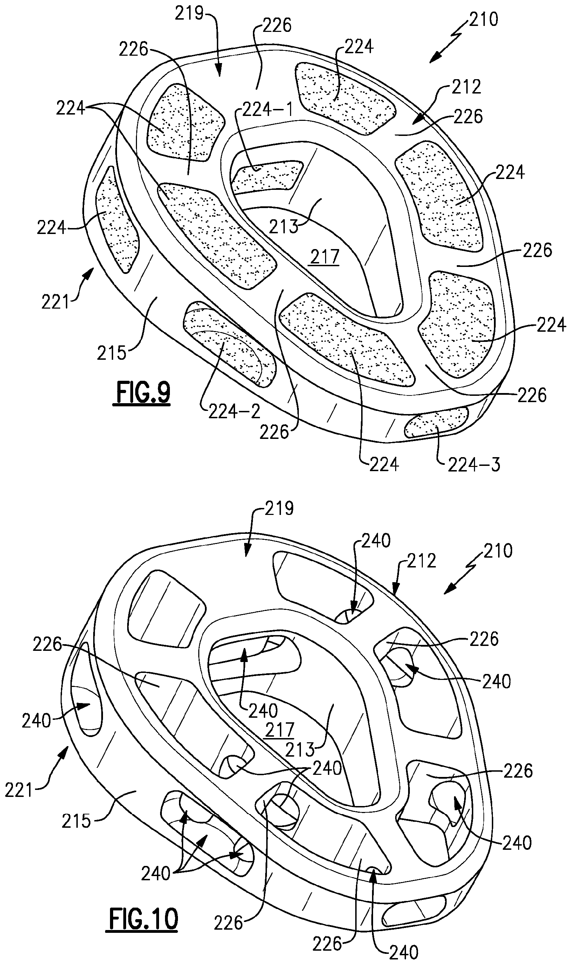

Figures 9, 10 and 11 illustrate yet another surgical implant 210. In this example not forming part of the invention, the surgical implant 210 is a wedge, such as an osteotomy wedge, that can be used to repair a bone deformity. For example, the surgical implant 210 can be implanted during an osteotomy procedure to repair a deformity associated with a knee, ankle, foot or other joint.

The surgical implant 210 includes a body 212 that can include any size and any shape. In one non-limiting embodiment, the body 212 extends between an inner wall 213 and an outer wall 215 that is spaced from the inner wall 213. The outer wall 215 may circumscribe the inner wall 213. A central bore 217 may extend through the body 212 inside of the inner wall 213. A top face 219 and a bottom face 221 of the body 212 may extend between the inner wall 213 and the outer wall 215.

One or more porous regions 224 may be formed in the body 212 of the surgical implant 210 (see Figure 9). Each porous region 224 includes a plurality of interconnected pores that establish areas of increased porosity that facilitate tissue ingrowth subsequent to implantation of the surgical implant 210 into bone. The central bore 217 may further facilitate tissue ingrowth. In one embodiment, at least one continuous porous region 224 extends continuously around the body 212 between the inner wall 213 and the outer wall 215. In other embodiments, some of the porous regions 224 may extend through the inner wall 213 (see, for example, the porous region 224-1) and/or the outer wall 215 (see, for example, the porous regions 224-2, 224-3).

In another non-limiting embodiment, one or more solid struts 226 connect between solid sections of the surgical implant 210. For example, the solid struts 226 may extend between solid portions of the inner wall 213 and the outer wall 215 and between solid portions of the top face 219 and the bottom face 221. The solid struts 226 increase the strength and stability of the body 212 of the surgical implant 210 near the porous regions 224. In one embodiment, the porous regions 224 are at least partially separated from one another by the solid struts 226.

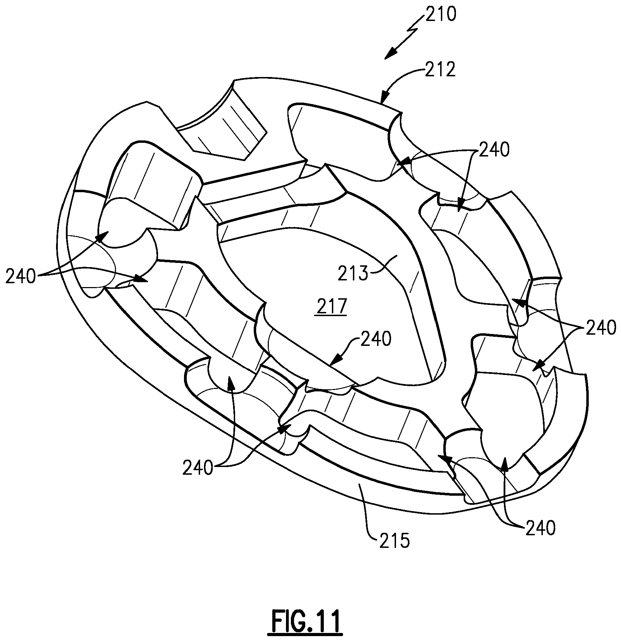

As best shown in Figures 10 and 11, which illustrate the surgical implant 210 with the porous regions 224 removed, vents 240, or openings, may be formed through both the inner wall 213 and the outer wall 215. Some of the vents 240 could also extend through the solid struts 226. In one embodiment, adjacent porous regions 224 of the surgical implant 210 connect with one another via the vents 240 to establish a continuous area of porosity distributed through the entirety of the body 212. Stated another way, the porous regions 224 may extend continuously through the body 212 to establish an uninterrupted network of interconnected pores inside the body 212. Therefore, the porous regions 224 may not be discrete regions of the body 212 that are isolated from other porous regions 224.

The porous regions of the various surgical implants of this disclosure are exemplary only and are not intended to limit this disclosure. The surgical implants may be designed to include more or less porosity within the scope of this disclosure.

Although the different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should also be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope and content of this disclosure.

## Claims

1. A surgical implant (10,110), comprising:
a body (12,112) that extends along a longitudinal axis between a drive head (14,114) and a tip (16,116) of the implant;
a non-porous thread (20,120) that wraps around said body (12,112) comprising a minor diameter (D1) and a major diameter (D2); and
a porous region (24,124) comprising a plurality of interconnected pores circumferentially extending about said body (12,112) between adjacent revolutions (25,125) of said non-porous thread (20), **characterized in that**
said body (12,112) including a cannulation (18,118) disposed about said longitudinal axis and extending from said tip (16,116) to said drive head (14,114); and **in that**
the porous region (24,124) extends from the minor diameter (D1) toward the cannulation (18,118).

2. The surgical implant (10,110) as recited in claim 1, wherein said surgical implant (10,110) is a suture anchor or an interference screw.

3. The surgical implant (10,110) as recited in claim 1, wherein said tip (16,116) and said drive head (14,114) are solid.

4. The surgical implant (10,110) as recited in claim 1, comprising a porous extension that extends from said porous region (24,124) to said cannulation (18,118).

5. The surgical implant (10,110) as recited in claim 1, comprising a vent that connects between said cannulation (18,118) and said porous region (24,124).

6. The surgical implant (10,110) as recited in claim 1, wherein said body (12,112) includes an inner wall (28,128) that circumscribes said cannulation (18,118), said inner wall (28,128) being solid.

7. The surgical implant (10,110) as recited in claim 1, comprising a solid strut (26,126) that connects between said non-porous thread (20,120) and said cannulation (18,118).

8. The surgical implant (10,110) as recited in claim 1, wherein said non-porous thread (20,120) is completely solid between said minor diameter and said major diameter.

9. The surgical implant (10,110) as recited in claim 1, wherein said body (12,112) and said porous region (24,124) are made of the same material.

10. The surgical implant (10,110) as recited in claim 1, wherein said porous region (24,124) is disposed in a portion of said body (12,112) that extends between said cannulation (18,118) and said non-porous thread (20,120).

11. The surgical implant (10,110) as recited in claim 1, wherein:
said cannulation (18,118) is circumscribed by an inner wall (28,128);
said non-porous thread (20,120) spirals about said body (12,112) between said drive head (14,114) and said tip (16,116), said non-porous thread (20,120) being completely non-porous between a minor diameter and a major diameter; and
a solid strut (26,126) that connects between said non-porous thread (20,120) and said inner wall (28,128).

12. The surgical implant (10,110) as recited in claim 11, comprising a vent formed through said inner wall (28,128), and said porous region (24,124) includes a porous extension that extends to said inner wall (28,128).

13. The surgical implant (10,110) as recited in claim 11, wherein said porous region (24,124) includes a height that extends across at least two of said revolutions (25,125) of said non-porous thread (20,120).

## Patentansprüche

1. Chirurgisches Implantat (10, 110), umfassend:
einen Körper (12, 112), der sich entlang einer Längsachse zwischen einem Antriebskopf (14, 114) und einer Spitze (16, 116) des Implantats erstreckt;
ein nicht poröses Gewinde (20, 120), das um den Körper (12, 112) herum verläuft, umfassend
einen kleineren Durchmesser (D1) und einen größeren Durchmesser (D2); und
einen porösen Bereich (24, 124), der eine Vielzahl von miteinander verbundenen Poren umfasst, die sich in Umfangsrichtung zwischen benachbarten Windungen (25, 125) des nicht porösen Gewindes (20) um den Körper (12, 112) herum erstrecken, **dadurch gekennzeichnet, dass**
der Körper (12, 112) eine Kanülierung (18, 118) beinhaltet, die um die Längsachse herum angeordnet ist und sich von der Spitze (16, 116) bis zu dem Antriebskopf (14, 114) erstreckt; und dass
der poröse Bereich (24, 124) sich von dem kleineren Durchmesser (D1) in Richtung der Kanülierung (18, 118) erstreckt.

2. Chirurgisches Implantat (10, 110) nach Anspruch 1, wobei das chirurgische Implantat (10, 110) ein Nahtanker oder eine Interferenzschraube ist.

3. Chirurgisches Implantat (10, 110) nach Anspruch 1, wobei die Spitze (16, 116) und der Antriebskopf (14, 114) massiv sind.

4. Chirurgisches Implantat (10, 110) nach Anspruch 1, eine poröse Verlängerung umfassend, die sich von dem porösen Bereich (24, 124) zu der Kanülierung (18, 118) erstreckt.

5. Chirurgisches Implantat (10, 110) nach Anspruch 1, eine Entlüftungsöffnung umfassend, die die Kanülierung (18, 118) mit dem porösen Bereich (24, 124) verbindet.

6. Chirurgisches Implantat (10, 110) nach Anspruch 1, wobei der Körper (12, 112) eine Innenwand (28, 128) beinhaltet, die die Kanülierung (18, 118) umgibt, wobei die Innenwand (28, 128) massiv ist.

7. Chirurgisches Implantat (10, 110) nach Anspruch 1, eine massive Strebe (26, 126) umfassend,
die das nicht poröse Gewinde (20, 120) mit der Kanülierung (18, 118) verbindet.

8. Chirurgisches Implantat (10, 110) nach Anspruch 1, wobei das nicht poröse Gewinde (20, 120) zwischen dem kleineren Durchmesser und dem größeren Durchmesser vollständig massiv ist.

9. Chirurgisches Implantat (10, 110) nach Anspruch 1, wobei der Körper (12, 112) und der poröse Bereich (24, 124) aus dem gleichen Material hergestellt sind.

10. Chirurgisches Implantat (10, 110) nach Anspruch 1, wobei der poröse Bereich (24, 124) in einem Abschnitt des Körpers (12, 112) angeordnet ist, der sich zwischen der Kanülierung (18, 118) und dem nicht porösen Gewinde (20, 120) erstreckt.

11. Chirurgisches Implantat (10, 110) nach Anspruch 1, wobei:
die Kanülierung (18, 118) von einer Innenwand (28, 128) umgeben ist;
das nicht poröse Gewinde (20, 120) zwischen dem Antriebskopf (14, 114) und der Spitze (16, 116) spiralförmig um den Körper (12, 112) verläuft, wobei das nicht poröse Gewinde (20, 120) zwischen einem kleineren Durchmesser und einem größeren Durchmesser vollständig nicht porös ist; und
eine massive Strebe (26, 126), die eine Verbindung zwischen dem nicht porösen Gewinde (20, 120) und der Innenwand (28, 128) herstellt.

12. Chirurgisches Implantat (10, 110) nach Anspruch 11, umfassend eine durch die Innenwand (28, 128) ausgebildete Entlüftungsöffnung, wobei der poröse Bereich (24, 124) eine poröse Verlängerung aufweist, die sich bis zu der Innenwand (28, 128) erstreckt.

13. Chirurgisches Implantat (10, 110) nach Anspruch 11, wobei der poröse Bereich (24, 124) eine Höhe aufweist, die sich über mindestens zwei der Windungen (25, 125) des nicht-porösen Gewindes (20, 120) erstreckt.

## Revendications

1. Implant chirurgical (10, 110), comprenant :
un corps (12, 112) qui s'étend le long d'un axe longitudinal entre une tête d'entraînement (14, 114) et une pointe (16, 116) de l'implant ;
un filet non poreux (20, 120) qui s'enroule autour dudit corps (12, 112) comprenant
un diamètre mineur (D1) et un diamètre majeur (D2) ; et
une région poreuse (24, 124) comprenant une pluralité de pores reliés entre eux s'étendant circonférentiellement autour dudit corps (12, 112) entre des révolutions adjacentes (25, 125) dudit filet non poreux (20), **caractérisé en ce que**
ledit corps (12, 112) comporte une canulation (18, 118) disposée autour dudit axe longitudinal et s'étendant de ladite pointe (16, 116) à ladite tête d'entraînement (14, 114) ; et **en ce que**
la région poreuse (24, 124) s'étend depuis le diamètre mineur (D1) vers la canulation (18, 118).

2. Implant chirurgical (10, 110) selon la revendication 1, dans lequel ledit implant chirurgical (10, 110) est une ancre de suture ou une vis d'interférence.

3. Implant chirurgical (10, 110) selon la revendication 1, dans lequel ladite pointe (16, 116) et ladite tête d'entraînement (14, 114) sont pleines.

4. Implant chirurgical (10, 110) selon la revendication 1, comprenant une extension poreuse qui s'étend de ladite région poreuse (24, 124) à ladite canulation (18, 118).

5. Implant chirurgical (10, 110) selon la revendication 1, comprenant un évent qui assure une liaison entre ladite canulation (18, 118) et ladite région poreuse (24, 124).

6. Implant chirurgical (10, 110) selon la revendication 1, dans lequel ledit corps (12, 112) comporte une paroi interne (28, 128) qui circonscrit ladite canulation (18, 118), ladite paroi interne (28, 128) étant pleine.

7. Implant chirurgical (10, 110) selon la revendication 1, comprenant une entretoise pleine (26, 126) qui assure la liaison entre ledit filet non poreux (20, 120) et ladite canulation (18, 118) .

8. Implant chirurgical (10, 110) selon la revendication 1, dans lequel ledit filet non poreux (20, 120) est complètement plein entre ledit diamètre mineur et ledit diamètre majeur.

9. Implant chirurgical (10, 110) selon la revendication 1, dans lequel ledit corps (12, 112) et ladite région poreuse (24, 124) sont faits du même matériau.

10. Implant chirurgical (10, 110) selon la revendication 1, dans lequel ladite région poreuse (24, 124) est disposée dans une partie dudit corps (12, 112) qui s'étend entre ladite canulation (18, 118) et ledit filet non poreux (20, 120).

11. Implant chirurgical (10, 110) selon la revendication 1, dans lequel :
ladite canulation (18, 118) est circonscrite par une paroi interne (28, 128) ;
ledit filet non poreux (20, 120) s'enroule en spirale autour dudit corps (12, 112) entre ladite tête d'entraînement (14, 114) et ladite pointe (16, 116), ledit filet non poreux (20, 120) étant complètement non poreux entre un diamètre mineur et un diamètre majeur ; et
une entretoise pleine (26, 126) qui assure la liaison entre ledit filet non poreux (20, 120) et ladite paroi interne (28, 128).

12. Implant chirurgical (10, 110) selon la revendication 11, comprenant un évent formé à travers ladite paroi interne (28, 128), et ladite région poreuse (24, 124) comporte une extension poreuse qui s'étend jusqu'à ladite paroi interne (28, 128).

13. Implant chirurgical (10, 110) selon la revendication 11, dans lequel ladite région poreuse (24, 124) comporte une hauteur qui s'étend sur au moins deux desdites révolutions (25, 125) dudit fil non poreux (20, 120).
